(19) Europäisches Patentamt
European Patent Office
Office européen des brevets

(11) Numéro de publication: **0 032 078**
**B1**

(12) **FASCICULE DE BREVET EUROPEEN**

(45) Date de publication du fascicule du brevet: **02.03.83**

(21) Numéro de dépôt: **80401753.1**

(22) Date de dépôt: **08.12.80**

(51) Int. Cl.³: **C 07 C 121/407,**
C 07 C 120/04

# (54) Procédé de préparation de cyanoacétates d'alkyle.

(30) Priorité: **20.12.79 FR 7931221**

(43) Date de publication de la demande:
**15.07.81 Bulletin 81/28**

(45) Mention de la délivrance du brevet:
**02.03.83 Bulletin 83/9**

(84) Etats contractants désignés:
**AT BE CH DE FR GB IT LI LU NL SE**

(56) Documents cités:
**FR - A - 1 302 365**
**FR - A - 1 383 366**
**FR - A - 1 447 526**
**FR - A - 1 450 529**
**FR - A - 2 021 641**
**FR - A - 2 357 533**
**US - A - 2 985 682**

(73) Titulaire: **RHONE-POULENC SPECIALITES CHIMIQUES**
**"Les Miroirs" 18, Avenue d'Alsace**
**F-92400 Courbevoie (FR)**

(72) Inventeur: **Desbois, Michel**
**526, Chemin du Bois**
**F-69140 - Rillieux (FR)**
Inventeur: **Soula, Gérard**
**33, rue Nungesser**
**F-69330 - Meyzieu (FR)**

(74) Mandataire: **Cazes, Jean-Marie et al,**
**RHONE-POULENC RECHERCHES Service Brevets Chimie et Polymères 25, quai Paul Doumer**
**F-92408 Courbevoie Cedex (FR)**

**EP 0 032 078 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de cyanoacétates d'alkyle

La présente invention a pour objet un procédé de préparation de cyanoacétates d'alkyle; elle concerne plus particulièrement la préparation de cyanoacétates d'alkyle par réaction de chloracétates d'alkyle avec du cyanure de sodium.

On connait dans l'art antérieur, le brevet français 1.447.516 qui décrit un procédé de préparation d'esters cyanacétiques qui consiste à faire réagir une quantité au moins équivalente d'un ester halogénoacétique avec un cyanure alcalin dans un solvant inerte, en présence d'acide cyanhydrique à une température comprise entre 20 et 250°C sous une pression comprise entre 1 et 6 atmosphères.

L'acide cyanhydrique n'est pas consommé pendant la réaction; il sert à supprimer les réactions secondaires de l'ester halogénoacétique sur l'ester cyané déjà produit avec formation de sous-produits. Selon ce brevet, il est particulièrement avantageux d'utiliser entre 1,1 et 6 moles d'ester halogénoacétique par mole de cyanure alcalin et entre 0,1 et 4 moles d'HCN par mole de cyanure alcalin. Les solvants cités sont principalement les alcools correspondant au composant alcoolique de l'ester mis en jeu.

Le principal inconvénient de ce type de procédé est que, pour avoir des vitesses de réaction compatibles avec une exploitation industrielle il est nécessaire de travailler au reflux du solvant. Cela pose alors de gros problèmes de sécurité puisque dans ces conditions de température l'acide cyanhydrique est sous forme vapeur.

On connait aussi dans l'art antérieur le brevet français 69.36380 publié sous le numéro 2.021.641 qui décrit un procédé de préparation d'esters cyanacétiques qui consiste à faire réagir du cyanure de sodium ou de potassium avec un ester halogénoacétique. La caractéristique de ce procédé réside dans le fait que la réaction est effectuée dans l'acétonitrile avec un excès de cyanure. Selon ce brevet, on peut opérer soit en l'absence d'eau, soit en présence d'eau. Selon les constatations de la demanderesse, il apparait que la mise en oeuvre de ce procédé en présence d'eau n'est envisageable industriellement que lorsque l'ester employé est dérivé d'un alcool tertiaire; en effet, dans le cas contraire, on observe une hydrolyse de la fonction ester dans un proportion qui nuit à l'économie du procédé. Mais, si l'on apère en l'absence d'eau, la réaction devient lente et la transformation est difficilement complète.

On voit donc que subsiste dans l'art antérieur le besoin d'un procédé non limité à un type d'ester déterminé, qu'il est possible de mettre en oeuvre à basse température dans des conditions de sécurité améliorées, avec une vitesse satisfaisante et des taux de transformation intéressants au plan industriel.

Les recherches de la demanderesse ont abouti à un tel procédé.

L'invention a donc pour objet un procédé de préparation de cyanoacétates d'alkyle par réaction de chloroacétate d'alkyle avec au moins un cyanure alcalin, les ions cyanure étant en excès, caractérisé en ce que l'on met en oeuvre la réaction dans un solvant inerte en présence d'au moins un agent séquestrant de formule générale:

$$N[-CHR_1-CHR_2-O-(CHR_3-CHR_4-O)_n-R_5]_3 \qquad (I)$$

dans laquelle n est un nombre entier supérieur ou égal à 0 et inférieur ou égal à 10 ($0 \leqslant n \leqslant 10$), $R_1$ $R_2$, $R_3$, $R_4$ identiques ou différents représentent un atome d'hydrogène ou un radical alkyle ayant de 1 à 4 atomes de carbone, $R_5$ représente un radical alkyle ou cycloalkyle ayant de 1 à 12 atomes de carbone, un radical phényle ou un radical de formule $-C_mH_{2m}-\phi$, ou $C_mH_{2m+1}-\phi-$, m étant compris entre 1 et 12.

L'invention repose sur le fait que l'agent séquestrant de formule (I) forme avec le cyanure alcalin un complexe dont la solubilité dans le solvant mis en oeuvre est meilleure que celle du cyanure alcalin seul.

Selon un mode de réalisation préférentiel de l'invention, on utilise au moins un agent séquestrant de formule (I) dans laquelle $R_1$, $R_2$, $R_3$, et $R_4$ représentent un atome d'hydrogène ou un radical méthyle, $R_5$ et n ayant la signification précédente.

Parmi ces derniers, on préfère encore plus particulièrement mettre en oeuvre les agents séquestrants pour lesquels n est supérieur ou égal à 0 et inférieur ou égal à 3, et pour lesquels $R_5$ représente un radical alkyle ayant de 1 à 4 atomes de carbone.

On peut citer:

— la tris(oxa-3-heptyl)amine de formule:

$$N-(CH_2-CH_2-O-C_4H_9)_3$$

— la tris(dioxa-3,6 heptyl)amine de formule:

$$N-(CH_2-CH_2-O-CH_2-CH_2-CH_3)_3$$

— la tris(trioxa-3,6,9 décyl)amine de formule:

$$N(CH_2—CH_2—O—CH_2—CH_2—O—CH_2CH_2—O—CH_3)_3$$

— la tris(dioxa-3,6 octyl)amine de formule:

$$N(CH_2—CH_2—O—CH_2—CH_2—O—C_2H_5)_3$$

— la tris(trioxa-3,6,9 undécyl)amine de formule:

$$N(CH_2—CH_2—O—CH_2—CH_2—O—CH_2—CH_2—O—C_2H_5)_3$$

— la tris(dioxa-3,6, nonyl)amine de formule:

$$N(CH_2—CH_2—O—CH_2—CH_2—O—C_3H_7)_3$$

— la tris(trioxa-3,6,9 dodécyl)amine de formule:

$$N(CH_2—CH_2—O—CH_2—CH_2—O—CH_2—CH_2—O—C_3H_7)_3$$

— la tris(dioxa-3,6 décyl)amine de formule:

$$N(CH_2—CH_2—O—CH_2—CH_2—O—C_4H_9)_3$$

— la tris(trioxa-3,6,9 tridécyl)amine de formule:

$$N(CH_2—CH_2—O—CH_2—CH_2—O—CH_2—CH_2—O—C_4H_9)_3$$

On peut encore citer:
— la tris(dioxa-3,6 méthyl-4 heptyl)amine de formule:

$$N(CH_2—CH_2—O—CHCH_3—CH_2—O—CH_3)_3$$

— la tris(dioxa-3,6 diméthyl-2,4 heptyl)amine de formule:

$$N(CH_2—CHCH_3—O—CHCH_3—CH_2—O—CH_3)_3.$$

Les amines utilisées dans le procédé selon l'invention sont connues en tant que telles dans l'art antérieur. C'est ainsi que le brevet français 1.302.365 cite l'obtention des amines tertiaires $N(CH_2—CH_2—O—CH_3)_3$ et $N(CH_2—CH_2—O—CH_2—CH_2—O—CH_3)_3$ comme sous produits de la synthèse des amines primaires et secondaires correspondantes, ces amines primaires et secondaires étant des produits intéressants comme intermédiaires en vue de la synthèse de substances pharmaceutiques, comme inhibiteurs de corrosion, comme intermédiaires en vue de la synthèse de produits chimiques intéressants en agriculture et comme émulsifiants. Il n'est pas inutile de souligner que le domaine d'application des composés obtenus dans le brevet 1.302.365 précité simultanément aux amines utilisées dans le procédé objet de la présente demande est totalement étranger au domaine de l'invention.

Le solvant mis en oeuvre est de préférence choisi parmi le groupe comprenant les alcools et les nitriles. On peut citer comme exemples, le méthanol, l'éthanol, les propanols, les butanols, l'acétonitrile et propionitrile. Parmi ceux-ci on préfère tout particulièrement utiliser l'acétonitrile.

Selon un mode particulier de l'invention on met en oeuvre la réaction en présence en outre d'acide cyanhydrique. Ce dernier n'affecte pas l'action de l'agent séquestrant tout en diminuant la formation de produits secondaires.

Le procédé selon l'invention est applicable à la réaction d'un chloroacétate d'alkyle de formule:

$$Cl—CH_2—COOR$$

dans laquelle R est un radical alkyle ayant un nombre d'atome de carbone compris entre 1 et 5.

Sont plus particulièrement concernés par le procédé selon l'invention, car permettant la synthèse des dyanoacétates les plus intéressants au plan industriel, les chloroacétates suivants: le chloroacétate de méthyle, le chloroacétate d'éthyle, le chloroacétate de tertiobutyle.

Les cyanures alcalins qui peuvent être utilisés dans le cadre du procédé selon l'invention sont principalement le cyanure de sodium et le cyanure de potassium.

Le cyanure alcalin est de préférence utilise en quantité telle que le rapport molaire du cyanure

alcalin au chloroacétate est supérieur ou égal à 1 et, encore plus préférentiellement, compris entre 1 et 3.

L'acide cyanhydrique, quand il est mis en oeuvre, est de préférence utilisé, sous forme liquide, en quantité telle que le rapport molaire de l'acide cyanhydrique au chloroacétate est supérieur à 0 et inférieur ou égal à 2. On préfère tout particulièrement mettre en oeuvre un rapport voisin de 1.

On utilise de préférence, l'agent séquestrant de formule (I) en quantité telle que le rapport molaire de l'agent séquestrant au chloroacétate est compris entre 0,001 et 0,1. Ce rapport est, encore plus préférentiellement, compris entre 0,01 et 0,05.

On opère de préférence à une température comprise entre environ −30°C et 60°C quand on n'utilise pas d'acide cyanhydrique et entre −30°C et +30°C dans le cas contraire; encore plus préférentiellement, on opère entre −5°C et +20°C.

On utilise de préférence une quantité de solvant telle que le nombre de moles de chloroacétate par litre de solvant est compris entre 0,5 et 5. Encore plus préférentiellement, on opère avec de 1 à 3 moles de chloroacétate par litre de solvant.

La réaction est généralement mise en oeuvre à la pression atmosphérique bien que des pressions supérieures ou inférieures à la pression atmosphérique ne soient pas exclues du domaine de l'invention.

Les composés obtenus selon le procédé selon l'invention, composés de formule NC $CH_2$—COOR où R à signification précédente, sont utiles, notamment comme intermédiaires de synthèse dans l'industrie pharmaceutique et dans l'industrie des matières plastiques.

Les agents séquestrants de formule I utilisés dans le procédé selon l'invention peuvent être préparés par condensation d'un sel de formule:

$$R_5—(O—CHR_4—CHR_3)_n—O—M$$

où $R_3$, $R_4$, $R_5$, et n ont la signification précédente et où M représente un atome de métal alcalin choisi parmi le sodium, le potassium et le lithium, soit sur une amine de formule générale:

$$N—(CHR_1—CHR_2—X)_3$$

dans laquelle $R_1$, et $R_2$ ont la signification précédente et X représent le chlore ou le brome, soit sur le chlorhydrate ou le bromhydrate correspondant.

Le rapport molaire sel de métal alcalin/amine est compris entre 3 et 5.

L'opération de condensation est réalisée à une température comprise entre 100 et 150°C pendant 1 à 15 heures en présence d'un solvant qui peut être par exemple le chlorobenzène ou de préférence le monoalkyléther d'éthylène glycol de formule:

$$R_5—(O—CHR_4—CHR_3)_n—OH.$$

On opère de préférence de telle sorte qu'on ait une solution contenant de 2 à 5 moles de sel de métal alcalin par litre de solvant.

Le mélange en fin de réaction contient principalement l'amine tertiaire de formule:

$$N—[CHR_1—CHR_2—O—(CHR_3—CHR_4—O)_nR_5]_3$$

mais contient aussi en faible proportion de l'amine secondaire correspondant:

$$HN—[CHR_1—CHR_2—O—(CHR_3—CHR_4—O)_n—R_5]_2$$

et des traces d'amine primaire:

$$H_2N—[CHR_1—CHR_2—O—(CHR_3—CHR_4—O)_n—R_5]$$

Les amines tertiaires, secondaires et primaires sont généralement respectivement dans le rapport 90 : 8 : 2 après distillation.

On peut utiliser dans le procédé selon l'invention directement le mélange ci-dessus obtenu après première distillation, c'est-à-dire contenant les trois types d'amines.

On préfère pour une meilleure mise en oeuvre de l'invention effectuer une distillation plus poussée de mélange ci-dessus afin d'obtenir une amine tertiaire sensiblement pure.

D'autres avantages et caractéristiques de l'invention apparaitront plus clairement à la lecture des exemples qui vont suivre qui ne sauraient en aucune manière être considérés comme une limitation de l'invention.

Exemple 1

Dans un réacteur double enveloppe parfaitement agité et muni d'un réfrigérant à boules, on introduit 1 l d'acétonitrile anhydre que l'on refroidit à 5°C. On introduit alors dans l'ordre 56,7 g (2,1

moles) d'acide cyanhydrique, 171,5 g (1,4 mole) de chloro-acétate d'éthyle et 14,6 g (0,04 mole) de tris(dioxa-3,6 octyl)amine.

Après homogénéisation du milieu, on introduit 137,2 g (2,8 moles/l) de cyanure de sodium sec en poudre. On agite alors fortement le mélange réactionnel pendant environ 8 heures et en gardant la température entre 0 et 10°C. On procède alors à une filtration des sels minéraux lesquels sont lavés ensuite 2 fois par 100 ml d'acétonitrile.

L'ensemble de la solution organique ainsi obtenue est alors distillée sous pression réduite. On recueille de cette manière 147,1 g de cyanoacétate d'éthyle soit un rendement de 93% par rapport au chloroacétate d'éthyle engagé avec un taux de transformation de 100%.

Exemples 2 A 11

On opère comme dans l'exemple 1 dans les conditions indiquées dans le tableau I. Le tableau II donne les résultats obtenus.

Essai comparatif 1

On opère comme dans l'exemple 7 mais sans ajouter d'agent séquestrant. Au bout de 8 heures, le taux de transformation n'est que de 5%.

Essai comparatif 2

On opère comme dans l'exemple 11 mais sans ajouter d'agent séquestrant. Au bout de 5 heures, le taux de transformation n'est que de 10%.

*PREPARATION DE LA TRIS(DIOXA-3,6 HEPTYL) AMINE*

— Préparation du méthoxy-2 éthanolate de sodium

Dans un ballon tricol d'un litre, muni d'un agitateur mécanique, d'un thermomètre, d'un réfrigérant, on introduit 380 g de méthanol-2 éthanol (5 moles). On ajoute 23 g de sodium (1 mole) en 3 heures en maintenant la température de mélange à 40°C.

— Synthèse de la tris(dioxa-3,6 heptyl)amine

Au mélange précédent, on ajoute 51,6 g de chlorhydrate de tris(chloro-2 éthyl)amine (soit 0,125 mole). On chauffe alors le mélange à reflux du méthoxy-2 éthanol (125°C) pendant 12 heures, puis on distille le solvant sous pression réduite. Le méthoxy-2 éthanolate de sodium en excès est neutralisé par addition de 11,6 cm$\alpha$ d'HCl aqueux (10 N). Le chlorure de sodium est filtré et la solution est distillée.

La tris(dioxa-3,6 heptyl)amine distille entre 165°C et 80°C sous 0,5 mmHg. On obtient 49 g de produit soit un rendement de 70%.

Les autres agents séquestrants utilisables dans le cadre du procédé selon l'invention peuvent être préparés par un procédé analogue.

TABLEAU I

| EX | chloroacétate d'éthyle g (mole(s)/litre) | cyanure de sodium g (mole(s)/litre) | HCN g (mole(s)/litre) | agent séquestrant nature (*) g (mole(s)/litre) | solvant nature 1 | température °C |
|---|---|---|---|---|---|---|
| 2 | 171,5 (1,4) | 98 (2) | 0 | TDA—2 14,6 (0,04) | $CH_3CN$ 1 | 50°C |
| 3 | 171,5 (1,4) | 83,3 (1,7) | 0 | TDA—2 14,6 (0,04) | $CH_3CN$ 1 | 50°C |
| 4 | 171,5 (1,4) | 88,2 (1,8) | 0 | TDA—2 14,6 (0,04) | $CH_3CN$ 1 | 0°C |
| 5 | 85,7 (0,7) | 49 (1) | 0 | TDA—2 7,2 (0,02 | $CH_3CN$ 1 | 30°C |
| 6 | 245 (2) | 98 (2) | 0 | TDA—1 32,5 (0,1) | $CH_3CN$ 1 | 20°C |

TABLEAU I (Suite)

| EX | chloroacétate d'éthyle g (mole(s)/litre) | cyanure de sodium g (mole(s)/litre) | HCN g (mole(s)/litre) | agent séquestrant nature (*) g (mole(s)/litre) | solvant nature 1 | température °C |
|---|---|---|---|---|---|---|
| 7 | 245 (2) | 98 (2) | 0 | TDA—1 32,5 (0,1) | $CH_3CN$ 1 | 0°C |
| 8 | 245 (2) | 98 (2) | 0 | TDA—1 32,5 (0,1) | $CH_3CN$ 1 | −20°C |
| 9 | 171,5 (1,4) | 98 (2) | 54 (2) | TDA—2 14,6 (0,04) | $CH_3CN$ 1 | 0°C |
| 10 | 171,5 (1,4) | 98 (2) | 13,5 (0,5) | TDA—2 14,6 (0,04) | $CH_3CH_2OH$ 1 | 20°C |
| 11 | 343 (2,8) | 166,6 (3,4) | 62,1 (2,3) | TDA—2 29,2 (0,08) | $CH_3CN$ 1 | 20°C |

(*) TDA—1 = tris[dioxa-3,6 heptyl]amine (*) TDA—2 = tris[dioxa-3,6 octyl]amine

TABLEAU II

| EX. | cyanoacétate d'éthyle obtenu g | taux de transformation chloroacétate d'éthyle en % | rendement par rapport au chloroacétate engagé en % | temps de réaction en heure |
|---|---|---|---|---|
| 2 | 114 | 100 | 72 | 1,75 |
| 3 | 118,6 | 100 | 75 | 2 |
| 4 | 115,5 | 100 | 73 | 10 |
| 5 | 55,4 | 100 | 70 | 5 |
| 6 | 162,7 | 100 | 72 | 5 |
| 7 | 169,5 | 100 | 75 | 8 |
| 8 | 101,7 | 60 | 75 | 5 |
| 9 | 150,3 | 100 | 95 | 10 |
| 10 | 137,6 | 100 | 87 | 10 |
| 11 | 297,4 | 100 | 94 | 5 |

**Revendications**

1. Procédé de préparation de cyanoacétates d'alkyle par réaction de chloroacétate d'alkyle avec au moins un cyanure alcalin, les ions cyanure étant en excès, caractérisé en ce que l'on met en oeuvre la réaction dans un solvant inerte en présence d'au moins un agent séquestrant de formule générale:

$$N[-CHR_1-CHR_2-O-(CHR_3-CHR_4-O)_n-R_5]_3 \qquad (I)$$

dans laquelle n est un nombre entier supérieur ou égal à 0 et inférieur ou égal à 10 ($0 \leqslant n \leqslant 10$), $R_1$ $R_2$, $R_3$, $R_4$ identiques ou différents représentent un atome d'hydrogène ou un radical alkyle ayant de 1 à 4 atomes de carbone, $R_5$ représente un radical alkyle ou cycloalkyle ayant de 1 à 12 atomes de carbone, un radical phényle ou un radical de formule $-C_mH_{2m}-\phi$, ou $C_mH_{2m+1}-\phi-$, m étant compris entre 1 et 12.

2. Procédé selon la revendication 1 caractérisé en ce que dans la formule (I) de l'agent séquestrant, $R_1$, $R_2$, $R_3$, et $R_4$ identiques ou différents représentent un atome hydrogène ou un radical méthyle.

3. Procédé selon la revendication 1 caractérisé en ce que dans la formule (I) de l'agent séquestrant, n est supérieur ou égal à 0 et inférieur ou égal à 5.

4. Procédé selon la revendication 1 caractérisé en ce que dans la formule (I) de l'agent séquestrant, $R_5$ représente un radical alkyle ayant de 1 à 4 atomes de carbone.

5. Procédé selon la revendication 1, caractérisé en ce que dans la formule (I) de l'agent séquestrant, $R_1$, $R_2$, $R_3$ et $R_4$ identiques ou différents représentent un atome d'hydrogène ou un radical méthyle, n est un nombre entier supérieur ou égal à 0 et inférieur ou égal à 3 et $R_5$ représente un radical alkyle ayant de 1 à 4 atomes de carbone.

6. Procédé selon la revendication 5 caractérisé en ce que l'agent séquestrant de formule (I) est la tris(dioxa-3,6 heptyl)amine de formule:

$$N-(CH_2-CH_2-O-CH_2-CH_2-O-CH_3)_3$$

7. Procédé selon la revendication 5 caractérisé en ce que l'agent séquestrant de formule I est la tris(dioxa-3,6 octyl) amine de formule:

$$N-(CH_2-CH_2-O-CH_2-CH_2-O-C_2H_5)_3$$

8. Procédé selon l'une quelconque des revendications 1 à 7 caractérisé en ce que la quantité d'agent séquestrant utilisée est telle que le rapport molaire de l'agent séquestrant au chloroacétate d'alkyle est comprise entre 0,001 et 0,1.

9. Procédé selon la revendication 8 caractérisé en ce que le rapport molaire de l'agent séquestrant au chloroacétate d'alkyle est compris entre 0,01 et 0,05.

10. Procédé selon la revendication 1 caractérisé en ce que le solvant inerte est choisi parmi le groupe comprenant les alcools et les nitriles.

11. Procédé selon la revendication 10 caractérisé en ce que le solvant inerte est choisi parmi le groupe comprenant le méthanol, l'éthanol, les propanols, les butanols, l'acétonitrile et le propionitrile.

12. Procédé selon la revendication 11 caractérisé en ce que le solvant inerte est l'acétonitrile.

13. Procédé selon l'une quelconque des revendications 1, 10, 11 et 12, caractérisé en ce que la quantité de solvant utilisée est telle que le nombre de moles de chloroacétate d'alkyle par litre de solvant est compris entre 0,5 et 5.

14. Procédé selon la revendication 13 caractérisé en ce que le nombre de moles de chloroacétate par litre de solvant est compris entre 1 et 3.

15. Procédé selon la revendication 1 caractérisé en ce que l'on met en oeuvre la réaction en présence en outre d'acide cyanhydrique.

16. Procédé selon la revendication 15, caractérisé en ce que la quantité d'acide cyanhydrique utilisée est telle que le rapport molaire de l'acide cyanhydrique au chloroacétate d'alkyle est supérieur à 0 et inférieur ou égal à 2.

17. Procédé selon la revendication 16, caractérisé en ce que le rapport molaire de l'acide cyanhydrique au chloroacétate d'alkyle est compris entre 1 et 1,5.

18. Procédé selon l'une quelconque des revendications précédentes caractérisé en ce que l'on met en oeuvre la réaction à une température comprise entre −30°C et +50°C.

19. Procédé selon la revendication 18 caractérisé en ce que la température est comprise entre −5°C et +20°C.

**Claims**

1. Process for the preparation of alkyl cyanoacetates by reaction of an alkyl chloroacetate with at least one alkali metal cyanide, the cyanide ions being in excess, characterised in that the reaction is

carried out in an inert solvent, in the presence of at least one sequestering agent of the general formula:

$$N—[CHR_1—CHR_2—O—(CHR_3—CHR_4—O)_n—R_5]_3 \qquad (I)$$

in which n is an integer which is greater than or equal to 0 and less than or equal to 10 ($0 \leqslant n \leqslant 10$), $R_1$, $R_2$, $R_3$ and $R_4$, which are identical or different, represent a hydrogen atom or an alkyl radical having from 1 to 4 carbon atoms, and $R_5$ represents an alkyl or cycloalkyl radical having from 1 to 12 carbon atoms, a phenyl radical or a radical of the formula: $—C_mH_{2m}—\phi$ or $C_mH_{2m+1}—\phi—$, m being between 1 and 12.

2. Process according to Claim 1, characterised in that, in the formula (I) of the sequestering agent, $R_1$, $R_2$, $R_3$ and $R_4$, which are identical or different, represent a hydrogen atom or a methyl radical.

3. Process according to Claim 1, characterised in that, in the formula (I) of the sequestering agent, n is greater than or equal to 0 and less than or equal to 5.

4. Process according to Claim 1, characterised in that, in the formula (I) of the sequestering agent, $R_5$ represents an alkyl radical having from 1 to 4 carbon atoms.

5. Process according to Claim 1, characterised in that, in the formula (I) of the sequestering agent, $R_1$, $R_2$, $R_3$ and $R_4$, which are identical or different, represent a hydrogen atom or a methyl radical, n is an integer which is greater than or equal to 0 or less than or equal to 3 and $R_5$ represents an alkyl radical having from 1 to 4 carbon atoms.

6. Process according to Claim 5, characterised in that the sequestering agent of the formula (I) is tris(3,6-dioxaheptyl)-amine of the formula:

$$N—(CH_2—CH_2—O—CH_2—CH_2—O—CH_3)_3.$$

7. Process according to Claim 5, characterised in that the sequestering agent of the formula (I) is tris-(3,6-dioxaoctyl)-amine of the formula:

$$N—(CH_2—CH_2—O—CH_2—CH_2—O—C_2H_5)_3.$$

8. Process according to any one of Claims 1 to 7, characterised in that the amount of sequestering agent used is such that the molar ratio of the sequestering agent to the alkyl chloroacetate is between 0.001 and 0.1.

9. Process according to Claim 8, characterised in that the molar ratio of the sequestering agent to the alkyl chloroacetate is between 0.01 and 0.05.

10. Process according to Claim 1, characterised in that the inert solvent is chosen from amongst the group comprising alcohols and nitriles.

11. Process according to Claim 10, characterised in that the inert solvent is chosen from amongst the group comprising methanol, ethanol, propanols, butanols, aceto-nitrile and propionitrile.

12. Process according to Claim 11, characterised in that the inert solvent is acetonitrile.

13. Process according to any one of Claims 1, 10, 11 and 12, characterised in that the amount of solvent used is such that the number of mols of alkyl chloroacetate per litre of solvent is between 0.5 and 5.

14. Process according to Claim 13, characterised in that the number of mols of chloroacetate per litre of solvent is between 1 and 3.

15. Process according to Claim 1, characterised in that the reaction is also carried out in the presence of hydrocyanic acid.

16. Process according to Claim 15, characterised in that the amount of hydrocyanic acid used is such that the molar ratio of the hydrocyanic acid to the alkyl chloroacetate is greater than 0 and less than or equal to 2.

17. Process according to Claim 16, characterised in that the molar ratio of the hydrocyanic acid to the alkyl chloroacetate is between 1 and 1.5.

18. Process according to any one of the preceding claims, characterised in that the reaction is carried out at a temperature of between –30°C and +50°C.

19. Process according to Claim 18, characterised in that the temperature is between –5°C and +20°C.

**Patentansprüche**

1. Verfahren zur Herstellung von Alkylcyanoacetaten durch Umsetzung von Alkylchloracetaten mit wenigstens einem Alkalicyanid, wobei die Cyanionen im Überschuß vorliegen, dadurch gekennzeichnet, daß man die Reaktion in einem inerten Lösungsmittel in Gegenwart wenigstens eines Sequestrierungsmittels der allgemeinen Formel:

$$N[—CHR_1CHR_2—O—(CHR_3—CHR_4—O)_n—R_5]_3 \qquad (I)$$

in der n eine ganze Zahl gleich oder größer 0 und kleiner oder gleich 10 ($0 \leqslant n \leqslant 10$) ist, $R_1$, $R_2$, $R_3$, $R_4$ gleich oder verschieden ein Wasserstoffatom oder einen Alkylrest mit 1 bis 4 Kohlenstoffatomen bedeuten und $R_5$ ein Alkyl- oder Cycloalkylrest mit 1 bis 12 Kohlenstoffatomen, ein Phenylrest oder ein Rest der Formel: $-C_mH_{2m}-\phi$ oder $C_mH_{2m+1}-\phi-$ ist, wobei m 1 bis 12 bedeutet.

2. Verfahren nach Anspruch 1, dadurch gekennzeichnet, daß in der Formel (I) des Sequestrierungsmittels $R_1$, $R_2$, $R_3$ und $R_4$ gleich oder verschieden, ein Wasserstoffatom oder einen Methylrest bedeuten.

3. Verfahren nach Anspruch 1, dadurch gekennzeichnet, daß in der Formel (I) des Sequestrierungsmittels n gleich oder größer 0 und kleiner oder gleich 5 ist.

4. Verfahren nach Anspruch 1, dadurch gekennzeichnet, daß in der Formel (I) des Sequestrierungsmittels $R_5$ ein Alkylrest mit 1 bis 4 Kohlenstoffatomen ist.

5. Verfahren nach Anspruch 1, dadurch gekennzeichnet, daß in der Formel (I) des Sequestrierungsmittels $R_1$, $R_2$, $R_3$, $R_4$ gleich oder verschieden, ein Wasserstoffatom oder einen Methylrest bedeuten, n eine ganze Zahl größer oder gleich 0 und kleiner oder gleich 3 und $R_5$ ein Alkylrest mit 1 bis 4 Kohlenstoffatomen ist.

6. Verfahren nach Anspruch 5, dadurch gekennzeichnet, daß das Sequestrierungsmittel der Formel (I) Tris(dioxa-3,6-heptyl)amin der Formel:

$$N(CH_2-CH_2-O-CH_2-CH_2-O-CH_3)_3$$

ist.

7. Verfahren nach Anspruch 5, dadurch gekennzeichnet, daß das Sequestrierungsmittel der Formel (I) das Tris(dioxa-3,6 octyl)amin der Formel:

$$N(CH_2-CH_2-O-CH_2-CH_2-O-C_2H_5)_3$$

ist.

8. Verfahren nach irgendeinem der Ansprüche 1 bis 7, dadurch gekennzeichnet, daß die Menge des verwendeten Sequestrierungsmittels derart ist, daß das Molverhältnis des Sequestrierungsmittels zum Alkylchloracetat zwischen 0,001 und 0,1 liegt.

9. Verfahren nach Anspruch 8, dadurch gekennzeichnet, daß das Molverhältnis des Sequestrierungsmittels zum Alkylchloracetat zwischen 0,01 und 0,05 liegt.

10. Verfahren nach Anspruch 1, dadurch gekennzeichnet, daß das inerte Lösungsmittel aus der Gruppe der Alkohole und Nitrile gewählt wird.

11. Verfahren nach Anspruch 10, dadurch gekennzeichnet, daß das inerte Lösungsmittel aus der Gruppe von Methanol, Äthanol, den Propanolen, den Butanolen, Acetonitril und Propionitril gewählt wird.

12. Verfahren nach Anspruch 11, dadurch gekennzeichnet, daß das inerte Lösungsmittel Acetonitril ist.

13. Verfahren nach irgendeinem der Ansprüche 1, 10, 11 und 12, dadurch gekennzeichnet, daß die Menge des verwendeten Lösungsmittels derart ist, daß die Zahl der Mole Alkylchloracetat pro Liter Lösungsmittel zwischen 0,5 und 5 liegt.

14. Verfahren nach Anspruch 13, dadurch gekennzeichnet, daß die Anzahl der Mole des Chloracetats pro Liter Lösungsmittel zwischen 1 und 3 liegt.

15. Verfahren nach Anspruch 1, dadurch gekennzeichnet, daß man die Reaktion zusätzlich in Gegenwart von Cyanwasserstoffsäure durchführt.

16. Verfahren nach Anspruch 15, dadurch gekennzeichnet, daß die Menge der verwendeten Cyanwasserstoffsäure derart ist, daß das Molverhältnis der Cyanwasserstoffsäure zum Alkylchloracetat größer als 0 und kleiner oder gleich 2 ist.

17. Verfahren nach Anspruch 16, dadurch gekennzeichnet, daß das Molverhältnis der Cyanwasserstoffsäure zum Alkylchloracetat zwischen 1 und 1,5 liegt.

18. Verfahren nach irgendeinem der vorangehenden Ansprüche, dadurch gekennzeichnet, daß man die Reaktion bei einer Temperatur zwischen $-30°C$ und $+50°C$ durchführt.

19. Verfahren nach Anspruch 18, dadurch gekennzeichnet, daß die Temperatur zwischen $-5°C$ und $+20°C$ liegt.